Europäisches Patentamt

European Patent Office

Office européen des brevets

(11) Veröffentlichungsnummer: **0 472 117 B1**

(12) **EUROPÄISCHE PATENTSCHRIFT**

(45) Veröffentlichungstag der Patentschrift: **08.11.95**

(51) Int. Cl.⁶: **C07F 9/09**, C07D 307/62

(21) Anmeldenummer: **91113708.1**

(22) Anmeldetag: **16.08.91**

(54) **Verfahren zur Herstellung von Kaliummagnesium-L-ascorbat-2-phosphat.**

(30) Priorität: **24.08.90 DE 4026787**

(43) Veröffentlichungstag der Anmeldung:
**26.02.92 Patentblatt 92/09**

(45) Bekanntmachung des Hinweises auf die
Patenterteilung:
**08.11.95 Patentblatt 95/45**

(84) Benannte Vertragsstaaten:
**CH DE FR GB LI NL**

(56) Entgegenhaltungen:
**EP-A- 0 275 198**
**EP-A- 0 308 918**
**EP-A- 0 388 869**

**CHEMICAL ABSTRACTS, Band 79, Nr. 11, 17.
September 1973, Columbus, Ohio, USAISHII,
SATORU et al. "3-phospho-L-ascorbate." Seite 480, Spalte 2, Zusammen-fassung-Nr. 66
743g**

**CHEMICAL ABSTRACTS, Band 98, Nr. 9, 28.
Februar 1983, Columbus, Ohio, USATAKEDA
CHEMICAL INDUSTRIES, LTD. "L-Ascorbic
acid phos- phates." Seite 684,Spalte 1, Zu-
sammen-fassung-Nr. 72 675v**

(73) Patentinhaber: **BASF Aktiengesellschaft
Carl-Bosch-Strasse 38
D-67063 Ludwigshafen (DE)**

(72) Erfinder: **Dobler, Walter, Dr.
Feuerbachstrasse 17
W-6900 Heidelberg (DE)**
Erfinder: **Voss, Hartwig, Dr.
Weinbietring 19
W-6710 Frankenthal (DE)**
Erfinder: **Balkenhohl, Friedhelm, Dr.
Trifelsring 37
W-6703 Limburgerhof (DE)**
Erfinder: **Paust, Joachim, Dr.
Ringstrasse 3
W-6708 Neuhofen (DE)**

CHEMICAL ABSTRACTS, Band 110, Nr. 18, 1. Mai 1989, Columbus, Ohio, USA SHO-JI,KAZUHISA et al. "Skin cosmetics containing ascorbyl phosphates andglycyrrhizinates." Seite 390, Spalte 1, Zusammen-fassung-Nr. 160 221r

CHEMICAL ABSTRACTS, Band 113, Nr. 17, 22. Oktober 1990, Columbus, Ohio, USAOKADA, TERUMI et al. "Preparation of crystalline ofL-ascorbic acid 2-phosphateester sodium salt." Seite 390, Spalte 2, Zusammen-fassung-Nr. 152 976n

**Beschreibung**

Die vorliegende Erfindung betrifft ein Verfahren zur Herstellung von Kaliummagnesium-L-ascorbat-2-phosphat, einem neuen Salz von L-Ascorbinsäure-2-phosphat mit sehr guten anwendungstechnischen Eigenschaften.

L-Ascorbinsäure (Vitamin C) ist ein lebenswichtiger Teil einer ausgewogenen menschlichen Ernährung und es hat sich eine empfohlene diätetische Verabreichung dieses Vitamins eingebürgert. Jedoch ist das Vitamin C das am wenigsten stabile Vitamin in Nahrungsmitteln, da es äußerst stark mit dem Luftsauerstoff reagiert. Es ist bekannt, daß man die Ascorbinsäure gegenüber Sauerstoff und Hitze stabiler machen kann, indem man sie in geeignete Derivate überführt. Dies ist besonderes wichtig für den neuerdings großen Einsatz von Vitamin C beim sogenannten Fischfarming.

Ascorbinsäuremonophosphat (ASMP) hat gegenüber der freien Ascorbinsäure (AS) folgende wesentliche Vorteile:

1) relativ hohe Oxidationsstabilität,
2) allgemeine Bioverfügbarkeit, da es durch Phosphatasen in Vivo und in Vitro zu Ascorbinsäure spaltbar ist (Dies wurde nachgewiesen z.B. am Meerschweinchen, an Broilern, an Ferkeln, Rhesusaffen und Fischen),
3) hohe Hitzebeständigkeit, und dadurch die Möglichkeit zum Arbeiten in Extrudern, sowie
4) hohe Hydrolysebeständigkeit.

Nachteilig bei fast allen bekannten Verfahren zur Herstellung von Ascorbinsäuremonophosphat ist, daß sie nur für ein Arbeiten in kleinem Maßstab geeignet sind, da für eine technische Durchführung die beschriebene Aufarbeitung viel zu aufwendig ist. Das Problem ist, daß das Wertprodukt von einem großen Überschuß an aus dem Phosphorylierungsverfahren stammenden anorganischen Salzen abgetrennt werden muß. So erhält man beispielsweise gemäß dem Verfahren gemäß DE-OS 27 19 303 bei der Phosphorylierung eine Reaktionslösung, die pro Äquivalent Wertprodukt etwa 4,5 Äquivalente KCl und 1,8 Äquivalente $K_3PO_4$, d.h. zusammen ca. 6 Äquivalente anorganische Salze enthält.

Einen Fortschritt gegenüber diesem Stand der Technik bedeutete das Verfahren gemäß der JP-A-63-77890 (Derwent AN88-136367). Diese JP-A beinhaltet ein Verfahren zur Reinigung von L-Ascorbat-2-phosphaten, das dadurch gekennzeichnet ist, daß man bei der Reinigung einer wäßrigen Lösung von L-Ascorbat-2-phosphaten die Verunreinigungen durch Elektrodialyse entfernt. Das L-Ascorbat-2-phosphat wird hierbei letztlich als Magnesiumsalz ausgefällt und isoliert. Nachteilig an diesem Verfahren ist, daß man entweder mit einem großen Magnesiumsalz-Überschuß arbeiten (vgl. Beispiel 1) oder aber zusätzlich mit großen Mengen an Kationenaustauschern arbeiten muß (vgl. Beispiele 2 und 3). Außerdem zeigt das bei diesem Verfahren als Reaktionsprodukt anfallende Magnesium-L-ascorbat-2-phosphat gewisse Nachteile bei der Isolierung, beispielsweise einen relativ hohen Filtrationswiderstand, Neigung zum Verklumpen beim Trocknen sowie Staubbildung bei der Weiterverwendung.

In der nicht vorveröffentlichten EP 388 869 A1 wird ein Verfahren zur Herstellung von Ascorbinsäure-2-phosphat durch Umsetzen von Ascorbinsäure oder Ascorbinsäurederivaten mit $POCl_3$ unter Aufrechterhalten eines pH-Wertes von etwa 8 bis 13,5 mittels KOH vorgeschlagen, bei dem man das anfallende Reaktionsgemisch mit der etwa stöchiometrischen Menge einer Magnesiumverbindung versetzt bis die Bildung von kristallinem $KMgPO_4$ beendet ist, dies abfiltriert und aus dem Filtrat das Ascorbinsäurephosphat in an sich bekannter Weise isoliert.

Es war die Aufgabe der Erfindung, die bei der Phosphorylierung von Isopropyliden-L-ascorbinsäure (IPAS) mit $POCl_3$ nach Abtrennen von Phosphorylierungshilfsstoffen und einem Teil der gebildeten anorganischen Salzes anfallende stark mit KCl verunreinigten Lösung von Kalium-L-ascorbat-2-phosphat auf noch vorteilhaftere Weise von Verunreinigungen zu befreien, und dabei das L-Ascorbat-2-phosphat in Form eines besser handhabbaren Salzes zu isolieren.

Es wurde nun überraschenderweise gefunden, daß man aus Ascorbat-2-phosphat-lösungen, die neben überschüssigem KCl nur etwa äquivalente Mengen an $MgCl_2$, bezogen auf Ascorbat-2-phosphat enthalten, mittels konventioneller Elektrodialyse überschüssiges Salz gut abtrennen kann, ohne daß größere Wertproduktverluste auftreten. Dabei verschiebt sich das Kationenverhältnis sehr stark zugunsten von $Mg^{2+}$, wodurch man ein Kalium-Magnesium-Mischsalz erhält und überschüssiges KCl nahezu selektiv abgereichert werden kann.

Es wurde weiterhin überraschenderweise gefunden, daß sich das bisher noch nicht beschriebene Kaliummagnesium-L-ascorbat-2-phosphat durch besonders vorteilhafte Produkteigenschaften auszeichnet. Es fällt aus Lösungen in gut kristalliner Form aus. Es besitzt dadurch extrem gute Filtrationseigenschaften und neigt nicht zur Verklumpung beim Trocknen (vgl. Beispiel 1). Es besitzt einen definierten Kristallwassergehalt und ist nicht hygroskopisch. Da es im Gegensatz zum reinen Magnesiumsalz nur 3 statt 5 Mol Kristallwasser pro Mol Ascorbat enthält, ist der Vitamin-C-Gehalt eines solchen Trockenpulvers höher.

Aufbauend auf diese Befunde wurde ein Verfahren entwickelt, das es erlaubt, die Reinigung von L-Ascorbat-2-phosphatlösungen durch Elektrodialyse vorteilhafter auszuführen und das L-Ascorbat-2-phosphat in Form eines besser handhabbaren Salzes zu isolieren.

Gegenstand der Erfindung ist daher ein Verfahren zur Herstellung von Kaliummagnesium-L-ascorbat-2-phosphat aus einer stark mit Kaliumchlorid verunreinigten wäßrigen Lösung von Kalium-L-ascorbat-2-phosphat, das dadurch gekennzeichnet ist, daß man die wäßrige Lösung von Kalium-L-ascorbat-2-phosphat mit soviel Magnesiumchlorid versetzt, daß die Lösung etwa 0,9 bis 1,3, vorzugsweise 0,95 bis 1,1 Äquivalente Magnesiumionen pro Äquivalent Kalium-L-ascorbat-2-phosphat enthält, in der Lösung befindliches Kaliumchlorid durch Elektrodialyse bei einem pH-Wert zwischen 5 und 8 so weit abreichert, daß das Äquivalenzverhältnis von $Mg^{2+}/K^+$ einen Wert zwischen 3,3 und 1,3 beträgt und das Kaliummagnesium-L-ascorbat-2-phosphat aus der verbleibenden Lösung in an sich bekannter Weise durch Kristallisation isoliert.

Das neue Verfahren ist ganz besonders geeignet zur Herstellung von Kaliummagnesium-L-ascorbat-2-phosphat aus einer wäßrigen Lösung von Kalium-L-ascorbat-2-phosphat, wie sie bei der Phosphorylierung von 5,6-Isopropyliden-L-ascorbinsäure mit Phosphoroxychlorid; anschließendes Ausfällen der überschüssigen Phosphorsäure mit Magnesiumchlorid als $KMgPO_4$; destillativem Entfernen der als Phosphorylierungshilfsmittel eingesetzten Base, Behandeln des Reaktionsgemisches mit Salzsäure zwecks Abspaltung der Isopropyliden Schutzgruppe und Abtrennen der beim anschließenden Abkühlen auskristallisierten Kaliumchlorids anfällt.

Aus verfahrenstechnischen Gründen empfiehlt es sich, daß man das Magnesiumchlorid bei dem erfindungsgemäßen Verfahren in Form einer wäßrigen Lösung einsetzt.

Besonders gute Ausbeuten erzielt man bei dem erfindungsgemäßen Verfahren, wenn man die wäßrige L-Ascorbat-2-phosphat-Lösung während der Elektrodialyse auf einen pH-Wert zwischen 6 und 7, einstellt. Besonders vorteilhaft ist es, den pH-Wert durch Zufügen von KOH auf den bevorzugten pH-Wert einzustellen. Kalium und Magnesium müssen in dem erfindungsgemäß hergestellten Mischsalz nicht genau in stöchiometrischem Verhältnis vorliegen. Das Kalium/ Magnesiumverhältnis in der Lösung, aus der das Mischsalz ausgefällt wird, ist mit dem des daraus erhaltenen kristallinen Produktes nahezu identisch. Optimale Ergebnisse bei der Isolierung von Ascorbinsäure-2-phosphat in Form eines Kaliummagnesiummischsalzes werden erzielt, wenn das Salz eine Zusammensetzung von etwa $K_{1\pm0,3}Mg_{1\pm0,15}$ Ascorbat-2-phosphat aufweist.

Ein Salz dieser Zusammensetzung erhält man bei dem erfindungsgemäßen Verfahren, wenn man aus der mit Magnesiumchlorid versetzten Lösung von Kalium-L-ascorbat-2-phosphat durch Elektrodialyse das Kaliumchlorid soweit abreichert, daß das Äquivalentverhältnis von $Mg^{2+}/K^+$ einen Wert zwischen 3,3 und 1,3 beträgt.

Ein Salz mit der stöchiometrischen Zusammensetzung KMg-Ascorbat-2-phosphat erhält man, wenn man die Lösung von Kalium-L-ascorbat-2-phosphat mit so viel Magnesiumchlorid versetzt, daß die Lösung 0,9 bis 1,3 Äquivalente Magnesiumionen pro Äquivalent L-Ascorbat-2-phosphat enthält und aus dieser Lösung durch Elektrodialyse das Kaliumchlorid so weit abreichert, daß in der Lösung ein Äquivalenzverhältnis von Magnesium zu Kalium von etwa 2 : 1 vorliegt.

Gegenstand der Erfindung ist weiterhin ein besonders vorteilhaftes Gesamtverfahren zur Herstellung von Kaliummagnesium-L-ascorbat-2-phosphat gemäß Anspruch 1, das dadurch gekennzeichnet ist, daß man

A) 5,6-Isopropyliden-L-ascorbinsäure unter Aufrechterhalten eines pH-Wertes von 8 bis 13,5 mittels KOH und in Gegenwart von einem tertiären Amin bei Temperaturen von -10 bis +25°C in einem geeigneten wäßrigen Lösungsmittel mit $POCl_3$ umsetzt,

B) das bei der Phosphorylierung anfallende Reaktionsgemisch ohne vorherige Ionenaustauscherbehandlung mit 90 bis 110 Mol-%, bezogen auf im Reaktionsgemisch vorhandenes anorganisches Phosphat an Magnesiumchlorid versetzt,

C) das auskristallisierende $KMgPO_4$ abtrennt,

D) das dabei erhaltene Filtrat mit HCl auf einen pH-Wert von etwa 7 einstellt und anschließend das tertiäre Amin zusammen mit einem Teil des Wassers destillativ entfernt,

E) das erhaltene Konzentrat mit HCl auf einen pH-Wert von etwa 1 einstellt, bei Temperaturen von 30 bis 40°C etwa 2 Stunden rührt und ggf.

F) die nach Abkühlen und Abtrennen des auskristallisierten Kaliumchlorids erhaltene Reaktionslösung mit soviel Magnesiumchlorid versetzt, daß die Lösung 0,9 bis 1,3 Äquivalente Magnesiumionen pro Äquivalent Ascorbat-2-phosphat enthält, die Lösung mit KOH auf einen pH-Wert von 6 bis 7 einstellt,

G) aus dieser Lösung mittels Elektrodialyse Kaliumchlorid abreichert bis in der Lösung bei einem pH-Wert von 6 bis 7 ein Äquivalenzverhältnis von Magnesium zu Kalium von 3,3 bis 1,3 : 1, vorzugsweise

4

etwa 2 : 1, vorliegt und

H) aus der erhaltenen wäßrigen Lösung durch Einengen und/oder Behandeln mit Methanol, Ethanol oder Aceton das so erhaltene Kaliummagnesium-L-ascorbat-2-phosphat isoliert.

Durch das erfindungsgemäße Verfahren werden große Vorteile bei der Herstellung von Salzen des L-Ascorbinsäure-2-phosphats erzielt.

1) Man erhält ein Salz mit deutlich besseren Handlungseigenschaften (vgl. Beispiel 1) und einem höheren Vitamin-C-gehalt.

2) Die bei der Aufarbeitung zu entfernende Salzfracht im Reaktionsgemisch wird erheblich vermindert, da überraschenderweise nur äquivalente Mengen an Magnesiumchlorid zugesetzt werden müssen. Hierdurch wird der für die Elektrodialyse notwendige Stromverbrauch stark herabgesetzt.

3) Ein zusätzlicher Einsatz von Ionenaustauschern, wie sie gemäß JP-AS 77890/88 (vgl. Beispiele 2 und 3) notwendig war, entfällt.

4) Beim Arbeiten unter den Vorzugsbedingungen (pH-Wert der Lösung während der Elektrodialyse 6-7) treten nur äußerst geringe Verluste an Wertprodukt auf.

5) Das nach der Behandlung mit HCl bzw. bei der Elektrodialyse anfallende Salz ist nur unwesentlich verunreinigtes Kaliumchlorid, welches ohne weitere Behandlung in die Düngemittelproduktion eingehen kann.

Zur Durchführung des vorteilhaften Gesamtverfahrens seien noch folgende Angaben gemacht.

Bei der Abreicherung von KCl aus einer stark mit KCl verunreinigten Lösung von Kalium-L-ascorbat-2-phosphat mittels Elektrodialyse ist der Wertproduktverlust nur dann minimal, wenn Magnesiumionen in etwa äquivalenten Mengen, bezogen auf Ascorbat-2-phosphat zugegen sind. Geringere Mengen führen zu deutlichen Wertproduktverlusten. Wesentlich größere Mengen führen zu schlechter kristallisierenden Salzen, zur Erhöhung des Stromverbrauchs, da auch sie wieder durch Elektrodialyse entfernt werden müssen, sowie aufgrund höherer Laufzeiten auch zu höheren Wertproduktverlusten. Durch gezielte Modifizierung des Herstellverfahrens mit Anwendung besonders vorteilhafter Elektrodialysebedingungen kann erreicht werden, daß sich nach der Elektrodialyse pro Äquivalent Ascorbat-2-phosphat nur etwa je 1/3 Äquivalent $K^+$ und 2/3 Äquivalente $Mg^{2+}$ im Austrag befinden, wobei der $Cl^-$-Gehalt bis auf weniger als 0,02 Äquivalente/kg ($\cong$ 0,07 Gew.-%) abgesenkt ist. Der Stromverbrauch ist beim erfindungsgemäßen Verfahren besonders niedrig. Die Produktverluste betragen nicht mehr als 1 bis 2 %. Einen großen Einfluß auf die Wertproduktverluste hat neben dem $Mg^{2+}$-Gehalt der pH-Wert des Reaktionsgemisches während der Elektrodialyse. Ein pH-Wert von etwa 6 bis 7 erwies sich diesbezüglich als besonders vorteilhaft.

Die Phosphorylierung von 5,6-Isopropyliden-L-ascorbinsäure erfolgt mit Vorteil nach dem Verfahren von Seib (vgl. DE-OS 27 19 303), d.h. durch Umsetzen mit $POCl_3$ in Gegenwart eines tertiären Amins in einem geeigneten wäßrigen Lösungsmittel bei Temperaturen von -10 bis 25°C, unter Aufrechterhalten eines pH-Wertes von etwa 8 bis 13,5, vorzugsweise 10 bis 13, durch Zugabe von KOH während der gesamten Phosphorylierungsreaktion.

Als tertiäre Amine sind solche geeignet, die mit dem Reaktionsgemisch mischbar und nicht flüchtig sind und eine Ionisationskonstante von weniger als etwa $10^7$ aufweisen. Genannt seien beispielsweise niedere Trialkylamine, wie Triethylamin, sowie cyclische Amine, wie Pyridin.

Die besten Ausbeuten werden mit Pyridin erhalten. Das Amin verwendet man in etwa der 5-fach molaren Menge, bezogen auf die Ascorbinsäure.

Besonders vorteilhaft gelingt die Phosphorylierung, wenn die Konzentration des Amins im Reaktionsgemisch etwa 1,5 bis 3 Mol, vorzugsweise 2,2 bis 2,6 Mol und die der Ascorbinsäure etwa 0,3 bis 0,6 Mol, vorzugsweise 0,4 bis 0,5 Mol pro Liter beträgt.

Als Lösungsmittel verwendet man mit Vorteil Wasser.

Im allgemeinen sollte als Reaktionstemperatur die niedrigste Temperatur angewendet werden, bei der das Reaktionsgemisch flüssig und bei der sich das tertiäre Amin nicht als separate Phase abtrennt. Geeignet sind Temperaturen von -10 bis +10°C.

Das bei der Phosphorylierung anfallende Reaktionsgemisch wird dann zur Abtrennung des überschüssigen anorganischen Phosphats direkt und ohne vorherige Ionenaustauscherbehandlung bei einem pH-Wert > 7 mit der etwa der Menge an überschüssigem anorganischem Phosphat äquivalenten Menge an $MgCl_2$, vorzugsweise einer wäßrigen Lösung von $MgCl_2$ versetzt.

Das auskristallisierende $KMgPO_4$ wird abgetrennt.

Das dabei erhaltene Filtrat wird dann mit HCl neutralisiert und unter vermindertem Druck die als Phosphorylierungshilfsmittel eingesetzte Base zusammen mit einem Teil des Wassers abdestilliert.

Die Isopropylidengruppe wird vorteilhaft an dieser Stelle des Verfahrens abgespalten, indem man die aufkonzentrierte Lösung mit konz. HCl auf einen pH-Wert von 1 ansäuert und das Reaktionsgemisch noch 1 bis 20, vorzugsweise etwa 1,5 bis 2,5 Stunden auf Temperaturen von etwa 10 bis 60°C, vorzugsweise 30 bis 40 °C unter Rühren und Abdestillieren von Aceton erhitzt. Diese Behandlung mit HCl hat neben der Abspaltung von Aceton den Vorteil, daß die bei der Reaktion mit $POCl_3$ gebildeten Ascorbatdi-, -tri und -polyphosphate zu Ascorbatmonophosphat hydrolysieren und daß ca. 1/3 des in der Lösung befindlichen KCl der wäßrigen Lösung durch Abkühlen in fester Form auskristallisieren. Dieses kann ohne weitere Reinigung zu Düngemitteln verarbeitet werden.

Das durch Abtrennen von auskristallisiertem KCl erhaltene Filtrat ist eine sehr gut geeignete Ausgangslösung für die erfindungsgemäße Herstellung von Kaliummagnesium-L-ascorbat-2-phosphat durch Elektrodialyse.

Diese Lösung wird zur Minimierung des Wertproduktverlustes zuerst mit der erforderlichen Menge $MgCl_2$ versetzt und dann auf einen pH-Wert von 5 bis 8, vorzugsweise 6 bis 7, eingestellt. Hierbei ist es von Vorteil, wenn auch nicht zwingend, die Einstellung des pH-Wertes mit wäßriger KOH vorzunehmen.

Pro Äquivalent Ascorbatmonophosphat sollten vor der Elektrodialyse 0,9 bis 1,3 Äquivalente, vorzugsweise 0,95 bis 1,1 Äquivalente $Mg^{2+}$-Ionen vorliegen.

Die Entsalzung von wäßrigen Salzlösungen mittels Elektrodialyse und entsprechende Elektrodialyseapparaturen sind an sich bekannt und z.B. in H. Strathmann "Trennung von molekularen Mischungen mit Hilfe synthetischer Membranen", Steinkopf Verlag, Darmstadt, 1979, S. 76 bis 86, und in D.S. Flett "Ion Exchange Membranes", Ellis Horwood, Chichester 1983, S. 179 bis 191, beschrieben.

Die Elektrodialyse bei der vorliegenden Erfindung wird zweckmäßig so durchgeführt, daß man zwischen zwei Elektroden alternierend Anionen- und Kationenaustauschermembranen parallel zueinander anordnet, die durch zwischengelegte Distanzrahmen gebildeten Kammern gegeneinander abdichtet und die KCl- und $MgCl_2$-haltige pH-Wert-eingestellte Produktlösung (im nachfolgenden auch als Diluat bezeichnet), durch diejenigen Kammern führt, die in Richtung der Anode durch eine Anionenaustauschermembran begrenzt werden, und eine wäßrige KCl-haltige Lösung (im nachfolgenden auch als Konzentrat bezeichnet) durch diejenigen Kammern führt, die in Richtung der Anode durch eine Kationenaustauschermembran begrenzt werden.

Von den Diluat- und Konzentratkammern durch die jeweils letzte Membran, vorzugsweise eine Kationenaustauschmembran, getrennt, befindet sich der Kathoden- und Anodenraum. An den Elektroden wird vorteilhaft während des Elektrodialyseprozesses eine elektrolythaltige Lösung vorbeigeführt, um entstehende Gase aus den Elektrodenkammern auszutragen. Zur sogenannten Elektrodenspülung verwendet man zweckmäßig eine wäßrige Natriumsulfatlösung mit einem Gehalt von ca. 1 bis 10 Gew.-%.

Als Ionenaustauschermembran sind handelsübliche Standardmembranen einsetzbar, die z.B. eine Dicke von 0,1 bis 1 mm, einen Porendurchmesser von 1 bis 30 $\mu$m oder eine gelartige Struktur, eine jeweilige Permselektivität von > als 0,9 und einen elektrischen Widerstand von etwa 5 $\Omega$ cm$^2$ (Desalination, Band 34, S. 77 bis 95 (1980)) aufweisen.

Die Anionenaustauschermembranen sind üblicherweise aus einem Matrixpolymer aufgebaut, das chemisch gebundene kationische Gruppen enthält, während die Kationenaustauschermembranen ein Matrixpolymer mit anionischen Gruppen aufweisen. Ionenaustauschermembranen der genannten Art sind z.B. die stark basisch bzw. stark sauren, unter den Bezeichnungen SELEMION® (Asahi Glass), NEOSEPTA® (Tokoyama Soda) oder IONAC® (Ionac Chemical Company) erhältlichen Membranen auf Polystyrolbasis.

Man elektrodialysiert beim erfindungsgemäßen Verfahren im allgemeinen bei einer Temperatur, die 100 °C nicht übersteigt, vorzugsweise bei 15 bis 80 °C, und mit einer Stromdichte, die in der Regel 3000 A/m$^2$ nicht übersteigt, vorzugsweise mit 10 bis 1000 A/m$^2$, bis die gewünschte $Cl^-$-Abreicherung sowie das gewünschte $Mg^{2+}/K^+$-Verhältnis erreicht ist. Die für den Ionentransport durch die Membranen erforderliche Gleichspannung richtet sich dabei nach den Leitfähigkeiten im Diluat und Konzentrat sowie dem Membranabstand, der durch die Distanzrahmenkonstruktion der Elektrodialysezelle gegeben ist.

Die Durchführung des Verfahrens kann sowohl kontinuierlich in mehreren hintereinander geschalteten Membranstapeln als auch diskontinuierlich durch Kreislaufführung der Flüssigkeitsströme mit Puffergefäßen oder durch Mischformen dieser Verfahrensarten ausgeführt werden.

Aus dem Austrag der Elektrodialyse kann dann das gewünschte Kaliummagnesium-L-ascorbat-2-phosphat durch Einengen und/oder Behandeln mit Lösungsmitteln, wie Methanol, Ethanol oder Aceton durch Kristallisation isoliert werden.

Mit Hilfe des erfindungsgemäßen Verfahrens kann Kaliummagnesium-L-ascorbat-2-phosphat auf sehr vorteilhafte Weise hergestellt und isoliert werden. Kaliummagnesium-L-ascorbat-2-phosphat ist ein neues Salz von L-Ascorbinsäure-2-phosphat, das sich sehr gut isolieren und handhaben läßt.

Beispiel 1

Zum Nachweis der vorteilhaften Eigenschaften des neuen Kaliummagnesium-L-ascorbat-2-phosphats gegenüber Kalium-L-ascorbat-2-phosphat und Magnesium-L-ascorbat-2-phosphat wurde der Filtrationswiderstand, die Verklumpung beim Trocknen und die Staubbildung von Kaliummagnesium-L-ascorbat-2-phosphaten der Formel $K_x Mg_y C_6 H_6 PO_9$ x 3 $H_2O$, d.h. also von Kaliummangesium-L-ascorbat-2-phosphaten mit unterschiedlichem K/Mg-Verhältnis mit den entsprechenden Eigenschaften des Kaliumsalzes (X = 3; Y = 0) bzw. des Magnesiumsalzes (X = 0; Y = 1,5) verglichen. Die Ergebnisse sind in der folgenden Tabelle dargestellt.

| Versuch | X | Y | Filtrations-widerstand | Verklumpen beim Trocknen | Staubbildung |
|---|---|---|---|---|---|
| a) | 0,00 | 1,50 | +++ | +++ | +++ |
| b) | 0,08 | 1,46 | +++ | +++ | +++ |
| c) | 0,17 | 1,42 | +++ | ++ | ++ |
| d) | 0,27 | 1,37 | ++ | ++ | ++ |
| e) | 0,36 | 1,32 | ++ | + | ++ |
| f) | 0,48 | 1,26 | + | + | + |
| g) | 0,61 | 1,20 | - | - | + |
| h) | 0,75 | 1,12 | - | - | + |
| i) | 0,83 | 1,09 | - | - | + |
| j) | 1,08 | 0,96 | - | - | + |
| k) | 1,20 | 0,86 | - | - | + |
| l) | 1,54 | 0,73 | + | + | - |
| m) | 1,86 | 0,57 | + | + | - |
| n) | 2,15 | 0,43 | + | ++ | - |
| o) | 3,00 | 0,00 | Produkt fällt als Öl an | | |

Die Versuche ergaben, daß Salze mit der Zusammensetzung $K_{1\pm0,3} Mg_{1\pm0,15}$-Ascorbat-2-phosphat die besten Produkteigenschaften aufweisen.

Bewertungsskala:

+ + +    sehr groß
+ +    groß
+    durchschnittlich
-    gering

Beispiel 2

Durchführung und Ergebnisse der Entsalzungen

Die Apparatur bestand aus einer Elektrodialyse-Zelle (ED-Zelle) und drei Kreisläufen (Diluat-, Konzentrat- und Elektrodenspülkreislauf). Jeder dieser Kreisläufe war mit einer Magnetkreiselpumpe, einem Wärmetauscher und einem Vorratsbehälter (1 bis 10 l) ausgerüstet und über Schläuche mit der ED-Zelle verbunden.

Die ED-Zelle hatte zwei Platinelektroden, die jeweils eine Fläche von 35 cm² aufwiesen. Die Elektrodenräume waren von den angrenzenden Konzentratkammern durch Nafion® Kationenaustauschermembranen (Firma Du Pont) abgetrennt. Zwischen den Elektrodenkammern befanden sich 11 Konzentrat- und 10 Diluatkammern in alternierender Anordnung. Die Kammern waren voneinander abwechselnd durch Selemion®AMV und Selemion CMV Membranen (Fa. Asahi Glass) getrennt. Alle Membranen hatten eine aktive Fläche von 37 cm². Die Membranabstände betrugen 0,5 mm. Zulauf und Ablauf der jeweiligen Lösungen wurde durch entsprechende Verbindungsbohrungen in den Dichtrahmen und Endplatten sowie Anschluß an die entsprechenden Pumpkreise erreicht.

Die Apparatur war mit einer Gleichstromversorgung mit Geräten zur Messung von Temperatur, pH-Wert, Spannung, Strom und Leitfähigkeit sowie mit einer pH-Wert-gesteuerten Säuredosierung ausgestattet. Die Entsalzungen erfolgten in Batch-Fahrweise.

Die Kreisläufe der Elektrodialyseapparatur wurden mit den folgenden Lösungen beschickt:

Diluat:

1 kg Syntheselösung mit der aus Tabelle 1 ersichtlichen Zusammensetzung, sowie eine Testlösung, die KCl und $MgCl_2$ in äquivalenter Menge enthielt.

Konzentrat: 1 kg einer wäßrigen ca. 0,25 %igen KCl-Lösung.

Elektrodenspüllösung: 2 kg einer 5 gew.-%igen $Na_2SO_4$-Lösung.

Die Lösungen wurden im Kreis über die ED-Zelle gepumpt und bei ca. 40°C mit einer Zellspannung bis zu 30 V sowie einem Elektrodialysestrom bis zu 3 A elektrodialysiert. Die Durchflußmengen für Diluat und Konzentrat betrugen ca. 1 kg/min.

Der Verlauf von Strom und Spannung ergab sich aus dem sich einstellenden Widerstand der ED-Anordnung. Unterhalb von 30 V Zellspannung war ein Strom von 3 A möglich, wurden 30 V aufgrund eines Anstiegs des Widerstands während der ED erreicht, ging der Strom entsprechend zurück.

Der Abbruch erfolgte nach Erreichen des gewünschten Entsalzungsgrades. Die Ergebnisse sind in der Tabelle 1 zusammengefaßt.

Tabelle 1

Ergebnisse der Elektrodialyse

| Beispiel | 2a (Vergleich) | | 2b (Vergleich) | | 2c | | 2d | | 2e | | 2f (Vergleich) | |
|---|---|---|---|---|---|---|---|---|---|---|---|---|
| | E* | A** | E* | A** | E* | A** | E* | A** | E* | A** | E* | A** |
| Elektrodialyse-zeit (h) | 2,25 | | 3,0 | | 2,25 | | 2,67 | | 2,5 | | 3,5 | |
| **Diluat** | | | | | | | | | | | | |
| Menge (g) | 1005 | 605 | 999 | 512 | 1054 | 692 | 1081 | 662 | 1014 | 629 | 1000 | 789 |
| Leitfähigkeit (mS/cm) | 121 | 8,2 | 221 | 14 | 185 | 22 | 190 | 14 | 180 | 25 | 157 | 0,6 |
| pH-Wert | 6,5 | 7,0 | 4,4 | 5,0 | 6,0 | 6,5 | 6,3 | 6,8 | 3,2 | 3,6 | 7,4 | 4,1 |
| $K^+$ (eq/kg) | 1,43 | 0,08 | 2,56 | 0,09 | 2,46 | 0,43 | 2,43 | 0,22 | 2,17 | 0,21 | 1 | <0,01 |
| $Mg^{2+}$ (eq/kg) | 0,63 | 0,80 | 0,53 | 0,77 | 0,82 | 1,15 | 1,07 | 1,48 | 0,90 | 0,90 | 1 | <0,01 |
| $Cl^-$ (eq/kg) | 1,35 | 0,01 | 2,48 | 0,01 | 2,09 | 0,02 | 2,43 | 0,01 | 2,20 | 0,03 | 2 | 0,01 |
| ASMP (eq/kg) | 0,77 | 0,98 | 0,68 | 0,98 | 0,84 | 1,27 | 0,86 | 1,37 | 0,87 | 1,29 | 0 | 0 |
| eq $Mg^{2+}$/eq $ASMP^{3-}$ | 0,82 | 0,81 | 0,78 | 0,79 | 0,98 | 0,91 | 1,24 | 1,08 | 1,03 | 0,70 | - | - |
| eq $Mg^{2+}$/eq $K^+$ | 0,44 | 10,0 | 0,21 | 8,6 | 0,33 | 2,7 | 0,44 | 6,7 | 0,41 | 4,29 | 1 | ca.1 |
| **Abreicherung im Diluat** | | | | | | | | | | | | |
| $K^+$ (%) | 96,6 | | 98,2 | | 88,5 | | 94,5 | | 94,0 | | >99,2 | |
| $Mg^{2+}$ (%) | 23,6 | | 25,5 | | 7,9 | | 15,3 | | 38,0 | | >99,2 | |
| $Cl^-$ (%) | 99,6 | | 99,8 | | 99,4 | | 99,7 | | 99,2 | | 99,6 | |
| $ASMP^{3-}$ (%) | 23,4 | | 26,1 | | 0,7 | | 2,4 | | 8,0 | | - | |

\* Einsatz
\*\* Austrag

Das Beispiel 2c erbrachte im Rahmen des erfindungsgemäßen Verfahrens das optimale Ergebnis.. Eingesetzt wurde eine Syntheselösung mit einem Äquivalenzverhältnis $Mg^{2+}$ zu $ASMP^{3-}$ von ca. 1 und einem pH-Wert von 6. Nach fast vollständiger $Cl^-$-Abreicherung (99,4 %) wird eine um den Faktor 1,5 aufkonzentrierte Lösung erhalten, die ein $Mg^{2+}$ zu $K^+$-Verhältnis von 2,7 aufweist und 0,18 eq/kg $PO_4^{3-}$ (aus der nicht quantitativen $KMgPO_4$-Fällung) enthält. Aus diesem Austrag wurde durch Einengen und Fällung mit Methanol ein Kalium-Magnesium-Mischsalz mit der Zusammensetzung $K_{1\pm0,3}Mg_{1\pm0,15}ASMP$ erhalten. Der Wertproduktverlust an $ASMP^{3-}$ betrug bei der Entsalzung nur ca. 1 %.

Die Vergleichsbeispiele 2a und 2b zeigen den Einfluß eines niedrigeren Äquivalenzverhältnisses $Mg^{2+}$ zu $ASMP^{3-}$ (0,8) im Einsatz; das Beispiel 2d den eines höheren (1,24) als in Beispiel 2c..

Im Fall der Vergleichsbeispiele 2a und 2b sind drastische Wertproduktverluste zu verzeichnen.

Das Beispiel 2e zeigt, daß der Einsatz einer Syntheselösung mit einem Äquivalenzverhältnis $Mg^{2+}$ zu $ASMP^{3-}$ von ca. 1 und einem auf 3,2 abgesenkten pH-Wert zu einer deutlichen Steigerung es $ASMP^{3-}$-Verlustes (von 0,7 auf 8 %) führt.

Das Vergleichsbeispiel 2f soll den überraschenden Effekt, nämlich die fast selektive KCl-Abtrennung aus einem $K^+$-$Mg^{2+}$-$Cl^-$-$ASMP^{3-}$-Gemisch (wie in Beispiel 2 c spezifiziert), gegenüber einer Lösung, die neben KCl und $MgCl_2$ kein $ASMP^{3-}$ enthält, verdeutlichen. Es zeigt sich nämlich, daß bei der Entsalzung einer gemischten KCl-$MgCl_2$-Lösung, beide Salze in gleicher Weise abgetrennt werden.

Beispiel 3

a) Herstellung von 5,6-Isopropyliden-ascorbinsäure (IPAS)

Zu 480 ml (6,55 Mol) Aceton wurden bei Temperaturen von -10 bis 0°C 43,1 g eines 24 %igen Oleums zugetropft. Anschließend wurden 120,6 g (0,68 Mol) pulvrige Ascorbinsäure zugegeben und die erhaltene Suspension 5,5 Stunden (h) bei 0°C gerührt. Anschließend kühlte man auf -10°C ab, filtrierte über eine Fritte, saugte die Mutterlauge ab und trocknete den Filterkuchen durch 2-stündiges Evakuieren (Wasserstrahlpumpe). Die Ausbeute betrug 146 g (98,6 % der Theorie).

b) Phosphorylierung mit $POCl_3$

Die gemäß a) erhaltenen 146 g IPAS (680 mmol) wurden in eine $O_2$-freie Lösung von 300 ml Pyridin in 1200 ml Wasser eingetragen und dabei durch Zudosieren einer 50 %igen wäßrigen KOH der pH-Wert der Lösung auf 8 gehalten (Dulcometer). Anschließend wurde der pH-Wert der Lösung mit KOH auf 13 eingestellt, unter Konstanthaltung des pH-Wertes bei 0-10°C langsam über einen Feindosierer 146,4 g (955 mmol) $POCl_3$ zudosiert, und das erhaltene Reaktionsgemisch noch 15 Minuten (min) nachgerührt.

c) Ausfällen von $KMgPO_4$

Das gemäß b) erhaltene Reaktionsgemisch wurde mit 69 g $MgCl_2 \cdot 6H_2O$ (340 mmol) in Form einer 15 %igen wäßrigen Lösung versetzt, dann auf Raumtemperatur (RT) gebracht, von den gebildeten Kristallen aus $KMg\,PO_4$ abgesaugt und letztere 2 x mit 200 ml Wasser gewaschen. Das Kristallisat hatte nach dem Trocknen (50°C, 100 mbar) die Zusammensetzung $KMg\,PO_4 \times 5H_2O$. Ausbeute 85 g (342 mmol).

d) Entfernen des Pyridins aus dem Reaktionsgemisch

Das gemäß c) erhaltene Filtrat wurde mit 37 %iger wäßriger HCl auf einen pH-Wert von 7 eingestellt und am Sambay-Verdampfer unter vermindertem Druck auf 1000 g eingeengt. Das erhaltene Kondensat enthielt über 99 % des Pyridins in wäßriger Lösung.

e) Abspaltung der Isopropyliden-Schutzgruppe und Ausfällen von KCl

Das gemäß d) erhaltene Konzentrat (1000 g), das bereits etwas festes KCl enthielt, wurde warm mit 116 g einer 37 %igen HCl auf einen pH-Wert von 1 gebracht und 2 h bei Temperaturen zwischen 30 und 40°C gerührt. Anschließend kühlte man das Reaktionsgemisch auf 0°C ab, rührte 30 min nach, filtrierte die gebildeten Kristalle ab und wusch diese 2 x mit 100 ml Eiswasser. Man erhielt so 171 g (1156 mmol) reines KCl.

f) Elektrodialyse

Das gemäß e) erhaltene Filtrat wurde mit 206 g $MgCl_2 \cdot 6H_2O$ (1020 mmol) in Form einer 15 %igen wäßrigen Lösung versetzt und mit KOH auf einen pH-Wert von 6 bis 7 eingestellt.

Reaktionsgemisch: 1667 g

Vitamin-C-Gehalt: 109,6 g C gesamt ($\cong$ 90,9 %, bezogen auf Ascorbinsäure

93,7 g C als Ascorbat-2-phosphat (77,7 %)

15,9 g C als Bis-[2,2'-ascorbat]phosphat (13,2 %).

Diese Lösung wurde unter den in Beispiel 2 genannten Bedingungen mittels Elektrodialyse in Batch-Fahrweise entsalzt.

Man erhielt hierbei 1095 g Reaktionsgemisch

enthaltend 108,1 g C gesamt ($\cong$ 89,6 %, bezogen auf Ascorbinsäure)

93,0 g C als Ascorbat-2-phosphat (77,1 %) und

15,1 g C als Bis-[2,2'-ascorbat]-phosphat (12,5 %).

Das entsalzte Reaktionsgemisch wurde auf 390 g eingedampft (Rotationsverdampfer) und sofort warm in 800 ml gerührtes Methanol getropft. Nach Abkühlen auf RT wurden die gebildeten Kristalle durch Absaugen der Mutterlauge isoliert, der Filterkuchen 2 x mit 150 ml Methanol gewaschen und im $N_2$-Strom getrocknet.

Man erhielt 230,0 g $K_{0,8}Mg\,C_6H_6PO_9 \times 3,5H_2O$; Vitamin-C-Gehalt = 46,9 % [HPLC]

enthaltend 107,5 g C-Gesamt ($\cong$ 89,1 %, bezogen auf Ascorbinsäure)

92,9 g C als Ascorbat-2-phosphat (77,1 %)

10

13,9 g C als Bis [2,2'-ascorbat]-phosphat (11,5 %).

Die Gesamtausbeute, bezogen auf eingesetzte Ascorbinsäure, betrug 89,1 % der Theorie über alle Stufen.

**Patentansprüche**

1.  Verfahren zur Herstellung von Kaliummagnesium-L-ascorbat-2-phosphat aus einer stark mit Kaliumchlorid verunreinigten wäßrigen Lösung von Kalium-L-ascorbat-2-phosphat, dadurch gekennzeichnet, daß man die wäßrige Lösung von Kalium-L-ascorbat-2-phosphat mit soviel Magnesiumchlorid versetzt, daß die Lösung etwa 0,90 bis 1,3 Äquivalente Magnesiumionen pro Äquivalent L-Ascorbat-2-phosphat enthält, in der Lösung befindliches Kaliumchlorid mittels Elektrodialyse bei einem pH-Wert zwischen 5 und 8 soweit abreichert, daß das Äquivalenzverhältnis von $Mg^{2+}/K^+$ einen Wert zwischen 3,3 und 1,3 beträgt und das Kaliummagnesium-L-ascorbat-2-phosphat aus der verbleibenden Lösung in an sich bekannter Weise durch Kristallisation isoliert.

2.  Verfahren zur Herstellung von Kaliummagnesium-L-ascorbat-2-phosphat gemäß Anspruch 1, dadurch gekennzeichnet, daß man als stark mit Kaliumchlorid verunreinigte wäßrige Lösung von Kalium-L-ascorbat-2-phosphat eine Lösung verwendet, wie sie bei der Phosphorylierung von 5,6-Isopropyliden-L-ascorbinsäure mit Phosphoroxychlorid; anschließendes Ausfällen der überschüssigen Phosphorsäure mit Magnesiumchlorid als $KMgPO_4$; destillativem Entfernen der als Phosphorylierungshilfsmitteleingesetzten Base, Behandeln des Reaktionsgemisches mit Salzsäure zwecks Abspaltung der Isopropyliden-Schutzgruppe und Abtrennen des beim Abkühlen der Lösung auskristallisierten Kaliumchlorids anfällt.

3.  Verfahren zur Herstellung von Kaliummagnesium-L-ascorbat-2-phosphat gemäß Anspruch 1, dadurch gekennzeichnet, daß man die wäßrige Lösung von Kalium-L-ascorbat-2-phosphat mit soviel Magnesiumchlorid versetzt, daß die Lösung 0,95 bis 1,10 Äquivalente Magnesiumionen pro Äquivalent L-Ascorbat-2-phosphat enthält.

4.  Verfahren zur Herstellung von Kaliummagnesium-L-ascorbat-2-phosphat gemäß Anspruch 1, dadurch gekennzeichnet, daß man eine wäßrige Lösung von Kalium-L-ascorbat-2-phosphat verwendet, die mit KOH auf einen pH-Wert von 6 bis 7 eingestellt wurde.

5.  Verfahren zur Herstellung von Kaliummagnesium-L-ascorbat-phosphat gemäß Anspruch 1, dadurch gekennzeichnet, daß man aus der mit Magnesiumchlorid versetzten Lösung von Kalium-L-ascorbat-2-phosphat durch Elektrodialyse das Kaliumchlorid so weit abreichert, daß in der Lösung ein Äquivalenzverhältnis $Mg^{2+}/K^+$ von etwa 2 vorliegt.

6.  Verfahren zur Herstellung von Kaliummagnesium-L-ascorbat-2-phosphat gemäß Anspruch 1, dadurch gekennzeichnet, daß man das Kaliummagnesium-L-ascorbat-2-phosphat aus der bei der Elektrodialyse verbleibenden Lösung durch Einengen und/oder Behandeln mit Methanol, Ethanol oder Aceton isoliert.

7.  Verfahren zur Herstellung von Kaliummagnesium-L-ascorbat-2-phosphat gemäß Anspruch 1, dadurch gekennzeichnet, daß man das Magnesiumchlorid in Form einer wäßrigen Lösung verwendet.

8.  Verfahren zur Herstellung von Kaliummagnesium-L-ascorbat-2-phosphat gemäß Anspruch 1, dadurch gekennzeichnet, daß man
    A) 5,6-Isopropyliden-L-ascorbinsäure unter Aufrechterhalten eines pH-Wertes von 8 bis 13,5 mittels KOH und in Gegenwart von einem tertiären Amin bei Temperaturen von -10 bis +25°C in einem geeigneten wäßrigen Lösungsmittel mit $POCl_3$ umsetzt,
    B) das bei der Phosphorylierung anfallende Reaktionsgemisch ohne vorherige Ionenaustauscherbehandlung mit 90 bis 110 Mol.-%, bezogen auf im Reaktionsgemisch vorhandenes anorganisches Phosphat an Magnesiumchlorid versetzt,
    C) das auskristallisierte $KMgPO_4$ abtrennt,
    D) das dabei erhaltene Filtrat mit HCl auf einen pH-Wert von etwa 7 einstellt und anschließend das tertiäre Amin und einen Teil des Wassers destillativ entfernt,
    E) das erhaltene Konzentrat mit HCl auf einen pH-wert von etwa 1 ansäuert, bei Temperaturen von 30 bis 40°C etwa 2 Stunden rührt,

F) die nach Abkühlen und Abtrennen des auskristallisierten Kaliumchlorids erhaltene Reaktionslösung mit soviel Magnesiumchlorid versetzt, daß die Lösung 0,9 bis 1,3 Äquivalente Magnesiumionen pro Äquivalent Ascorbat-2-phosphat enthält, die Lösung mit KOH auf einen pH-Wert von 6 bis 7 einstellt,

G) aus dieser Lösung mittels Elektrodialyse Kaliumchlorid abreichert bis in der Lösung bei einem pH-Wert von 6 bis 7 ein Äquivalenzverhältnis $Mg^{2+}/K^+$ von 3,3 bis 1,3 : 1 vorliegt und

H) aus der erhaltenen wäßrigen Lösung durch Einengen und/oder Behandeln mit Methanol, Ethanol oder Aceton das so erhaltene Kaliummagnesium-L-ascorbat-2-phosphat isoliert.

**Claims**

1. A process for preparing potassium magnesium L-ascorbate 2-phosphate from an aqueous solution of potassium L-ascorbate 2-phosphate which is heavily contaminated with potassium chloride, which comprises adding to the aqueous solution of potassium L-ascorbate 2-phosphate sufficient magnesium chloride for the solution to contain about 0.90 to 1.3 equivalents of magnesium ions per equivalent of L-ascorbate 2-phosphate, reducing the concentration of potassium chloride in the solution by electrodialysis at a pH of from 5 to 8 until the $Mg^{2+}/K^+$ equivalent ratio is from 3.3 to 1.3, and isolating the potassium magnesium L-ascorbate 2-phosphate from the remaining solution by crystallization in a conventional manner.

2. A process for preparing potassium magnesium L-ascorbate 2-phosphate as claimed in claim 1, wherein the aqueous solution of potassium L-ascorbate 2-phosphate which is heavily contaminated with potassium chloride is one resulting from the phosphorylation of 5,6-isopropylidene-L-ascorbic acid with phosphorus oxychloride, subsequent precipitation of the excess phosphoric acid as $KMgPO_4$ with magnesium chloride, removal by distillation of the base employed in the phosphorylation, treatment of the reaction mixture with hydrochloric acid to eliminate the isopropylidene protective group, and removal of the potassium chloride which crystallizes on cooling the solution.

3. A process for preparing potassium magnesium L-ascorbate 2-phosphate as claimed in claim 1, wherein sufficient magnesium chloride is added to the aqueous solution of potassium L-ascorbate 2-phosphate for the solution to contain from 0.95 to 1.10 equivalents of magnesium ions per equivalent of L-ascorbate 2-phosphate.

4. A process for preparing potassium magnesium L-ascorbate 2-phosphate as claimed in claim 1, wherein the aqueous solution of potassium L-ascorbate 2-phosphate has been adjusted to a pH of from 6 to 7 with KOH.

5. A process for preparing potassium magnesium L-ascorbate 2-phosphate as claimed in claim 1, wherein the concentration of potassium chloride in the solution of potassium L-ascorbate 2-phosphate which has been mixed with magnesium chloride is reduced by electrodialysis until the $Mg^{2+}/K^+$ equivalent ratio is about 2.

6. A process for preparing potassium magnesium L-ascorbate 2-phosphate as claimed in claim 1, wherein the potassium magnesium L-ascorbate 2-phosphate is isolated from the solution remaining after electrodialysis by concentration and/or treatment with methanol, ethanol or acetone.

7. A process for preparing potassium magnesium L-ascorbate 2-phosphate as claimed in claim 1, wherein the magnesium chloride is used in the form of an aqueous solution.

8. A process for preparing potassium magnesium L-ascorbate 2-phosphate as claimed in claim 1, wherein
A) 5,6-isopropylidene-L-ascorbic acid is reacted with $POCl_3$ in a suitable aqueous solvent while maintaining a pH of from 8 to 13.5 using KOH and in the presence of a tertiary amine at from -10 to +25 °C,
B) from 90 to 110 mol %, based on the inorganic phosphate present in the reaction mixture, of magnesium chloride is added to the reaction mixture from the phosphorylation without previous treatment with ion exchanger,
C) the crystallized $KMgPO_4$ is separated off,

EP 0 472 117 B1

D) the resulting filtrate is adjusted to a pH of about 7 with HCl and subsequently distilled to remove the tertiary amine and part of the water,

E) the resulting concentrate is acidified to a pH of about 1 with HCl and stirred at from 30 to 40°C for about 2 hours,

F) sufficient magnesium chloride is added to the solution resulting after cooling and removal of the crystallized potassium chloride for the solution to contain from 0.9 to 1.3 equivalents of magnesium ions per equivalent of ascorbate 2-phosphate, and the solution is adjusted to a pH of from 6 to 7 with KOH,

G) the concentration of potassium chloride in this solution is reduced by electrodialysis until the $Mg^{2+}/K^+$ equivalent ratio at a pH of from 6 to 7 is from 3.3 to 1.3 : 1 and

H) the resulting potassium magnesium L-ascorbate 2-phosphate is isolated from the resulting aqueous solution by concentration and/or treatment with methanol, ethanol or acetone.

## Revendications

1. Procédé de préparation de L-ascorbate-2-phosphate de potassium et de magnésium à partir d'une solution aqueuse de L-ascorbate-2-phosphate de potassium fortement souillée de chlorure de potassium, caractérisé en ce que l'on additionne la solution aqueuse de L-ascorbate-2-phosphate de potassium de la quantité nécessaire de chlorure de magnésium pour que la solution contienne environ 0,90 à 1,3 équivalent d'ions magnésium par équivalent de L-ascorbate-2-phosphate, on élimine le chlorure de potassium qui se trouve dans la solution, par électrodialyse à un pH compris entre 5 et 8, dans une mesure telle que le rapport des équivalents $Mg^{2+}/K^+$ s'élève à une valeur comprise entre 3,3 et 1,3, et on isole le L-ascorbate-2-phosphate de potassium et de magnésium de la solution restante, de façon connue en soi, par cristallisation.

2. Procédé de préparation de L-ascorbate-2-phosphate de potassiumet et de magnésium selon la revendication 1, caractérisé en ce que l'on utilise, comme solution aqueuse de L-ascorbate-2-phosphate de potassium fortement souillée de chlorure de potassium, une solution qui a été obtenue par phosphorylation d'acide 5,6-isopropylidène-L-ascorbique avec de l'oxychlorure de phosphore, suivie de précipitation de l'acide phosphorique en excès avec du chlorure de magnésium sous forme de KMgPO$_4$, élimination par distillation de la base utilisée comme agent auxiliaire de phosphorylation, traitement du mélange réactionnel par de l'acide chlorhydrique afin d'éliminer le groupement isopropylidène de protection et séparation du chlorure de potassium qui se sépare par cristallisation lors du refroidissement de la solution.

3. Procédé de préparation de L-ascorbate-2-phosphate de potassium et de magnésium selon la revendication 1, caractérisé en ce que l'on additionne la solution aqueuse de L-ascorbate-2-phosphate de potassium de la quantité nécessaire de chlorure de magnésium pour que la solution contienne de 0,95 à 1,10 équivalent d'ions magnésium par équivalent de L-ascorbate-2-phosphate.

4. Procédé de préparation de L-ascorbate-2-phosphate de potassium et de magnésium selon la revendication 1, caractérisé en ce que l'on utilise une solution aqueuse de L-ascorbate-2-phosphate de potassium qui a été réglée à un pH de 6 à 7 avec KOH.

5. Procédé de préparation de L-ascorbate-2-phosphate de potassium et de magnésium selon la revendication 1, caractérisé en ce que, dans la solution de L-ascorbate-2-phosphate de potassium additionnée de chlorure de magnésium, on élimine le chlorure de potassium par électrodialyse dans une mesure telle qu'un rapport des équivalents $Mg^{2+}/K^+$ d'environ 2 soit présent dans la solution.

6. Procédé de préparation de L-ascorbate-2-phosphate de potassium et de magnésium selon la revendication 1, caractérisé en ce que l'on isole le L-ascorbate-2-phosphate de potassium et de magnésium de la solution qui reste à la suite de l'électrodialyse par concentration et/ou traitement par du méthanol, de l'éthanol ou de l'acétone.

7. Procédé de préparation de L-ascorbate-2-phosphate de potassium et de magnésium selon la revendication 1, caractérisé en ce que l'on utilise le chlorure de magnésium sous forme d'une solution aqueuse.

13

8. Procédé de préparation de L-ascorbate-2-phosphate de potassium et de magnésium selon la revendication 1, caractérisé en ce que

A) on fait réagir de l'acide 5,6-isopropylidène-L-ascorbique avec $POCl_3$ en maintenant un pH de 8 à 13,5 au moyen de KOH et en présence d'une amine tertiaire, à une température de -10 à +25°C, dans un solvant aqueux approprié,

B) on additionne le mélange réactionnel résultant de la phosphorylation, sans traitement préalable par un échangeur d'ions, de 90 à 110% en moles, par rapport au phosphate inorganique présent dans le mélange réactionnel, de chlorure de magnésium,

C) on sépare $KMgPO_4$ qui se déplace par cristallisation,

D) on règle le filtrat ainsi obtenu, avec du HCl, à un pH d'environ 7, puis on élimine par distillation l'amine tertiaire et une partie de l'eau,

E) on acidifie le concentré obtenu, avec du HCl, à un pH d'environ 1, on agite pendant 2 h environ à une température de 30 à 40°C,

F) on additionne la solution réactionnelle, obtenue après refroidissement et séparation du chlorure de potassium qui se sépare par cristallisation, de la quantité de chlorure de magnésium nécessaire pour que la solution contienne de 0,9 à 1,3 équivalent d'ions magnésium par équivalent d'ascorbate-2-phosphate, on règle la solution à un pH de 6 à 7 avec KOH,

G) on élimine le chlorure de potassium dans cette solution, par électrodialyse à un pH de 6 à 7, jusqu'à ce qu'un rapport des équivalents $Mg^{2+}/K^+$ de 3,3 à 1,3 soit présent dans la solution, et

H) on isole de la solution aqueuse obtenue, par concentration et/ou traitement par du méthanol, de l'éthanol ou de l'acétone, le L-ascorbate-2-phosphate de potassium et de magnésium ainsi obtenu.